Europäisches Patentamt

European Patent Office

Office européen des brevets

(19)

(11) Veröffentlichungsnummer: **0 179 421 B1**

(12) **EUROPÄISCHE PATENTSCHRIFT**

(45) Veröffentlichungstag der Patentschrift: **29.05.91**

(21) Anmeldenummer: **85113292.8**

(22) Anmeldetag: **19.10.85**

(51) Int. Cl.5: **A61K 31/57**, //(A61K31/57, 31:565,31:557)

(54) **Antigestagene, Glukokortikoide und Prostaglandine zur Geburtseinleitung oder für den Schwangerschaftsabbruch.**

(30) Priorität: **22.10.84 DE 3438994**

(43) Veröffentlichungstag der Anmeldung:
**30.04.86 Patentblatt 86/18**

(45) Bekanntmachung des Hinweises auf die Patenterteilung:
**29.05.91 Patentblatt 91/22**

(84) Benannte Vertragsstaaten:
**AT BE CH DE FR GB IT LI LU NL SE**

(56) Entgegenhaltungen:
**EP-A- 0 081 882**
**EP-A- 0 139 608**
**FR-A- 2 247 256**

(73) Patentinhaber: **SCHERING AKTIENGESELL-SCHAFT Berlin und Bergkamen**
**Müllerstrasse 170/178 Postfach 65 03 11**
**W-1000 Berlin 65(DE)**

(72) Erfinder: **Elger, Walter, Dr.**
**Schorlemerallee 12 B**
**W-1000 Berlin 33(DE)**
Erfinder: **Beier, Sybille, Dr.**
**Uhlandstrasse 121**
**W-1000 Berlin 31(DE)**

## Beschreibung

Die Erfindung betrifft ein Kombinationserzeugnis zur gemeinsamen Verwendung zur Geburtseinleitung oder für den Schwangerschaftsabbruch, enthaltend ein Prostaglandin (PG), ein Antigestagen (AG) und ein Glukokortikoid (GK) sowie die Verwendung von Prostaglandinen zur Geburtseinleitung oder für den Schwangerschaftsabbruch in Kombination mit Antigestagenen und Glukokortikoiden.

Um Gefahren für Mutter und/oder Kind abzuwenden, ist es manchmal erforderlich, eine Geburt künstlich einzuleiten oder eine Schwangerschaft vorzeitig zu beenden.

Dafür stehen chirurgische Techniken und pharmakologische Methoden zur Verfügung.

Eine günstige pharmakologische Methode ist die vaginale oder intramuskuläre Applikation von Prostaglandinen, die im Falle des Schwangerschaftsabbruchs im 1. oder 2. Schwangerschaftstrimester (Contraception 1983, Vol. 27, 51 - 60, und Int. J. Gynaecol. Obstet. 1982, Vol. 20, 383 - 386) vorgenommen wird.

Vorteile der Prostaglandine sind ihre einfache Anwendbarkeit und ihre Einsatzmöglichkeit über einen langen Zeitraum der Schwangerschaft. Als Nachteile der Prostaglandine sind akute Nebenwirkungen wie Schmerzen und Übelkeit zu nennen; außerdem liegt die Erfolgsrate im Falle des Schwangerschaftsabbruchs in fortgeschrittenen Phasen der Schwangerschaft auch bei einer längeren Dauer der Prostaglandinbehandlung nicht über 90 %.

Eine andere Möglichkeit der Beendigung einer Schwangerschaft besteht in der Applikation eines Antigestagens (Med. et Hyg. 1982, Vol. 40, 2087 - 2093). Antigestagene sind besser verträglich als Prostaglandine, haben aber eine größere Latenz und individuelle Variabilität des Wirkungseintritts im Vergleich mit den Prostaglandinen.

Sie lösen in Tiermodellen, in denen muskuläre Wirkungen am Uterus die entscheidende Rolle spielen, Aborte mit einer Latenz von mehreren Tagen aus. Einige sonst sehr wirksame Antigestagene sind in diesen Modellen auch bei den höchsten geprüften Dosen nur bei einem Teil der Tiere wirksam.

In der deutschen Patentanmeldung P 33 37 450.3 wird beschrieben, daß PG-typische und AG-typische Nachteile vermieden werden, wenn man Prostaglandine (PG) und Antigestagene (AG) gemeinsam für den Schwangerschaftsabbruch verwendet. Hierbei können die Gewichtsmengen an Prostaglandin und Antigestagen bei gemeinsamer Anwendung im Vergleich zu den üblichen Mengen stark herabgesetzt werden, wobei die Erfolgsrate der Schwangerschaftsabbrüche sogar noch gesteigert werden kann.

Es wurde nun gefunden, daß überraschenderweise eine weitere Steigerung der Erfolgsrate dadurch erreicht wird, daß man zusätzlich ein Glukokortikoid verabreicht. Als sehr günstig erweist es sich dabei, daß diese Steigerung gerade in den Fällen, in denen der Prostaglandin-Zusatz zu einem Antigestagen nicht so eine große Verbesserung der Abortrate erbracht hat, besonders stark ist.

Man hat mit diesem Dreikomponenten-Erzeugnis aus Prostaglandin, Antigestagen und Glukokortikoid ein Mittel zum therapeutischen Abbruch der Gravidität in allen Stadien der Schwangerschaft oder zur Geburtseinleitung mit höchsten Erfolgsraten zur Verfügung. Es ermöglicht besser standardisierbare Behandlungsverfahren, die die klinische Anwendung erleichtern.

Durch das erfindungsgemäße Erzeugnis wird die Zeit, die nach Behandlungsbeginn bis zur Ausstoßung verstreicht, durch eine zuverlässigere und raschere Aktivierung der ruhenden Muskulatur des graviden Uterus, deutlich verkürzt.

Durch die hohe Wirksamkeit der erfindungsgemäßen Dreikomponten-Kombination und die vergleichsweise kurzen Behandlungszeiten bis zum Therapieerfolg werden ausreichende therapeutische Effekte daher mit vergleichsweise niedrigen AG- und PG-Dosen erzielt.

Antigestagen und Glukokortikoid werden vorzugsweise getrennt und gleichzeitig, Antigestagen/Glukokortikoid und Prostaglandin vorzugsweise getrennt, gleichzeitig und/oder zeitlich abgestuft (sequential) oder auch Prostaglandin, Antigestagen und Glukokortikoid zusammen in einerDosiseinheit, in einem Gewichtsverhältnis von Prostaglandin zu Glukokortikoid von im wesentlichen 1 : 10 bis 1 : 3000 und Prostaglandin zu Antigestagen von im wesentlichen 1 : 10 bis 1 : 10000 verwendet.

Als für die erfindungsgemäße Verwendung geeignete Prostaglandine kommen alle für den Schwangerschaftsabbruch geeignete Prostaglandine infrage; das sind insbesondere Prostaglandine der E- und F-Reihe. Beispielsweise genannt seien:

Prostaglandin $E_2$,

Prostaglandin $F_{2\alpha}$'

Prostaglandin E-Derivate, wie

16-Phenoxy-$\omega$-17,18,19,20-tetranor-$PGE_2$-methylsulfonylamid (Sulproston),

16,16-Dimethyl-trans-$\Delta^2$-$PGE_1$-methylester (Gemeprost),

9-Deoxo-16,16-dimethyl-9-methylen-$PGE_2$ (Metenenprost),

2

Prostaglandin F-Derivate, wie
15-Methyl-PG $F_{2\alpha}$-methylester,
(5Z,13E)-(9R,11R,15R)-9-Chlor-11,15-dihydroxy-16,16-dimethyl-5,13-prostadiensäure (DE-OS 29 50 027),
(5Z,13E)-(9R,11R,15R)-11,15-Dihydroxy-9-fluor-16-phenoxy-17,18,19,20-tetranor-5,13-prostadiensäure (DE-OS 31 26 924),
(5Z,13E)-(9R,11R,15R)-11,15-Dihydroxy-16,16-dimethyl-9-fluor-5,13-prostadiensäure (DE-OS 31 26 924),
(5Z,13E)-(9R,11R,15R)-9-Brom-11,15-dihydroxy-16-phenoxy-17,18,19,20-tetranor-5,13-prostadiensäure (DE-OS 31 48 743),
(5Z,13E)-(9R,11R,15R)-9-Brom-11,15-dihydroxy-16,16-dimethyl-5,13-prostadiensäure (DE-OS 31 48 743).

Die Prostaglandine werden in Mengen eingesetzt, die deutlich unterhalb der sonst für den Schwangerschaftsabbruch üblichen Mengen liegen. Die gemäß vorliegender Erfindung anzuwendende Menge hängt u.a. vom Hormonspiegel, vom Alter der Gravidität und der Art der Applikation ab. Bei der Verwendung von Sulproston als Prostaglandin wird man in der Regel mit 0,02 bis 3,0 mg pro Tag auskommen. Die Applikation kann zum Beispiel lokal, topisch, enteral oder parenteral erfolgen. Bei der intramuskulären Injektion bzw. intravenösen Infusion werden beispielsweise Mengen von etwa 1,0 bis 3,0 mg Sulproston pro Tag benötigt. Bei der lokalen Applikation, beispielsweise extraamnial oder intravaginal, werden etwa 0,02 bis 0,5 mg Sulproston pro Tag eingesetzt. Eine Dosiseinheit Prostaglandin enthält 0,02 bis 0,5 mg Sulproston bzw. eine biologisch äquivalente Menge eines anderen Prostaglandins Für die topische Anwendung können auch transdermale Systeme wie Hautpflaster eingesetzt werden. Anstelle von Sulproston können erfindungsgemäß biologisch äquivalente Mengen von anderen Prostaglandinen eingesetzt werden.

Als Antigestagene kommen alle Verbindungen infrage, die eine starke Affinität zum Gestagenrezeptor (Progesteronrezeptor) besitzen und dabei keine eigene gestagene Aktivität zeigen. Als kompetitive Progesteronantagonisten kommen beispielsweise folgende Steroide infrage:
$11\beta$-[(4-N,N-Dimethylamino)-phenyl]-$17\beta$-hydroxy-$17\alpha$-propinyl-4,9(10)-estradien-3-on und
$11\beta$-[(4-N,N-Dimethylamino)-phenyl]-$17\beta$-hydroxy-18-methyl-$17\alpha$-propinyl-4,9(10)-estradien-3-on und
$11\beta$-[(4-N,N-Dimethylamino)-phenyl]-$17a\beta$-hydroxy-$17a\alpha$-propinyl-D-homo-4,9(10),16-estratrien-3-on
(Europäische Patentanmeldung 82400025.1 - Veröffentlichungs-Nr. 0 057 115); ferner
$11\beta$-p-Methoxyphenyl-$17\beta$-hydroxy-$17\alpha$-ethinyl-4,9(10)-estradien-3-on (Steroids 37 (1981) 361 - 382);
$11\beta$-(4-N,N-Dimethylaminophenyl)-$17\alpha$-hydroxy-$17\beta$-(3-hydroxy propyl)-$13\alpha$-methyl-4,9-gonadien-3-on
(Deutsche Patentanmeldung 33 21826)
$11\beta$-[(4-N,N-Dimethylamino)-phenyl]-$17\beta$-hydroxy-$17\alpha$-(3-hydroxyprop-1-(Z)-enyl)-4,9(10)-estradien-3-on
(Deutsche Patentanmeldung P 33 47 126.6).

Die Antigestagene werden gemäß vorliegender Erfindung in Mengen eingesetzt, die in der Regel unterhalb der sonst für den Schwangerschaftsabbruch üblichen Mengen liegen.

Im allgemeinen wird man mit 5 - 200 mg $11\beta$[(4-N,N-Dimethylamino)-phenyl]-$17\beta$-hydroxy-$17\alpha$-(3-hydroxy-prop-1-(Z)-enyl)-4,9(10)-estradien-3-on oder $11\beta$-[(4-N,N-Dimethylamino)-phenyl]-$17\alpha$-hydroxy-$17\beta$-(3-hydroxypropyl)-$13\alpha$-methyl-4,9-gonadien-3-on pro Tag oder einer biologisch äquivalenten Menge eines anderen Antigestagens auskommen.

Als Glukokortikoide kommen alle wirksamen Kortikoide infrage. Eine Zusammenstellung der üblichen Kortikoide findet man in E. Schröder, C. Rufer, R. Schmiechen "Pharmazeutische Chemie", Georg Thieme Verlag, Stuttgart (1982) S. 424. Beispielsweise genannt seien Prednison, Prednisolon, Fluocortolon, Triamcinolon, Methylprednisolon, Predniyliden, Paramethason, Dexamethason, Betamethason, Beclometason oder Fluprednyliden.

Die Dosierung der Glukokortikoide liegt beim Menschen bei 0,3 bis 100 mg. Im allgemeinen wird man mit 0,3 bis 10 mg Dexamethason pro Tag oder einer biologisch äquivalenten Menge eines anderen Glukokortikoids (z.B. 5 bis 100 mg Prednison pro Tag) auskommen.

Die Antigestagene und Glukokortikoide können zum Beispiel lokal, topisch, enteral oder parenteral appliziert werden.

Für die bevorzugte orale Applikation kommen insbesondere Tabletten, Dragées, Kapseln, Pillen, Suspensionen oder Lösungen infrage, die in üblicher Weise mit den in der Galenik gebräuchlichen Zusätzen und Trägersubstanzen hergestellt werden können. Für die lokale oder topische Anwendung kommen beispielsweise Vaginalzäpfchen oder transdermale Systeme wie Hautpflaster infrage.

Eine Dosiseinheit Antigestagen enthält 5 - 200 mg Wirkstoff. Eine Dosiseinheit Glukokortikoid enthält 0,3 - 100 mg Wirkstoff.

Die gemeinsame Behandlung mit Prostaglandin, Antigestagen und Glukokortikoid erfolgt in der Regel über 1 bis 4, vorzugsweise 1 bis 2 Tage, wobei Antigestagen und Glukokortikoid vorzugsweise getrennt und gleichzeitig und Prostaglandin vorzugsweise getrennt und gleichzeitig oder zeitlich abgestuft (sequential) mit Antigestagen und Glukokortikoid appliziert werden können.

Bei der Sequenztherapie werden zunächst Antigestagen und Glukokortikoid über 1 bis 3 Tage und anschließend das Prostaglandin allein oder Prostaglandin und Antigestagen/ Glukokortikoid gemeinsam über 24 Stunden appliziert.

Die drei Wirkstoffe können auch kombiniert in einer Dosiseinheit in ein und demselben Vehikel (z.B. ölige Lösung wie ein Benzylbenzoat-Rizinusöl-Gemisch) appliziert werden. Die nachstehenden Beispiele sollen die galenische Formulierung erläutern.

## Beispiel 1

## Zusammensetzung einer gefriergetrockneten Sulproston-Formulierung pro Ampulle

| | | |
|---|---|---|
| 0,1 | mg | Sulproston |
| 5,0 | mg | Polyvinylpyrrolidon (K-Wert = 15 - 18) |
| 1,95 | mg | Tris(hydroxymethyl)aminomethan-hydrochlorid |
| | | (Tremetamol · HCl) (aus 1,5 mg Tremetamol und |
| | | 1 N Salzsäure) |
| 7,05 | mg | |
| ======= | | |

Zur Dosierung und Anwendung wird der Inhalt der Ampulle mit isotonischer Kochsalzlösung aufgelöst für die intramuskuläre Injektion bzw. intravenöse Infusion oder extraamniale Applikation.

Herstellung der Trockensubstanz

Sulproston wird durch Zugabe einer eisgekühlten Lösung des Polyvinylpyrrolidons und Tremetamols in destilliertem Wasser in Lösung gebracht. Durch Zugabe von 1 N Salzsäure wird unter starker Kühlung der pH-Wert der Lösung auf 5,0 eingestellt. Anschließend wird die Lösung auf das erforderliche Volumen aufgefüllt. Nach Filtration über ein Membranfilter wird die Lösung in Ampullen dosiert.

Die Lösung wird dann durch Eintauchen der Ampullen in eine Aceton/Trockeneis-Kältemischung eingefroren und sofort in einer vorgekühlten Gefriertrockenanlage ca. 48 Stunden gefriergetrocknet. Nach Abschluß der Gefriertrocknung werden die Ampullen sofort zugeschmolzen.

Beispiel 2

Zusammensetzung einer Folie mit Sulproston für die
vaginale Applikation

 0,1  mg  Sulproston
 19,6  mg  Hydroxypropylcellulose
 0,32 mg  Polyoxyethylenpolyoxypropylenpolymeres
       (Pluronic F 68$^{(R)}$)
 ―――――――

 20,02 mg

 ========

Die Folie hat eine Länge von 3 cm.

Beispiel 3

Zusammensetzung einer Folie mit Sulproston für die
buccale Applikation

 0,3  mg  Sulproston
 9,16 mg  Hydroxypropylcellulose
 9,16 mg  Cellulosefasern
 0,15 mg  Polyoxyethylenpolyoxypropylenpolymeres
       (Pluronic F 68$^{(R)}$)
 ―――――――

 18,77 mg

 ========

Die Fläche der Folie ist 1,2 x 1,2 cm.

Beispiel 4

Zusammensetzung einer Tablette mit Sulproston zur
vaginalen Applikation

   0,1 mg  Sulproston
238,9 mg  Laktose
110,0 mg  mikrokristalline Cellulose
  1,0 mg  Magnesiumstearat
350,0 mg
========


Beispiel 5

Zusammensetzung einer weiteren Tablette mit
11ß-/̄(4-N,N-Dimethylamino)-pheny_l7̄-17ß-hydroxy-17α-
(3-hydroxy-prop-1-(Z)-enyl)-4,9(10)-estradien-3-on
zur oralen Applikation

 10,0 mg  11ß-/̄(4-N,N-Dimethylamino)-pheny_l7̄-17ß-hydroxy-
           17α-(3-hydroxy-prop-1-(Z)-enyl-4,9(10)-estradien-
           3-on
140,5 mg  Laktose
 69,5 mg  Maisstärke
  2,5 mg  Polyvinylpyrrolidon 25
  2,0 mg  Aerosil
  0,5 mg  Magnesiumstearat
225,0 mg
========

Beispiel 6

Zusammensetzung einer Tablette mit Dexamethason zur oralen Applikation

     0,050 mg  Dexamethason
    76,515 mg  Maisstärke USP XVI
    36,000 mg  Laktose
     6,000 mg  Talkum
     1,400 mg  Gelatine, weiß
     0,024 mg  Nipagin M$^{(R)}$ (p-Hydroxybenzoesäuremethylester)
     0,011 mg  Nipasol M$^{(R)}$ (p-Hydroxybenzoesäurepropylester)
   120,000 mg

   ==========

Beispiel 7

Zusammensetzung einer weiteren Tablette mit 11ß-/[4-N,N-Dimethylamino)-phenyl/-17α-hydroxy-17ß-(3-hydroxypropyl)-13α-methyl-4,9-gonadien-3-on zur oralen Applikation

   10,0 mg  11ß-/[4-N,N-Dimethylamino)-phenyl/-17α-hydroxy-17ß-(3-hydroxypropyl)-13α-methyl-4,9-gonadien-3-on
  140,5 mg  Laktose
   69,5 mg  Maisstärke
    2,5 mg  Polyvinylpyrrolidon 25
    2,0 mg  Aerosil
    0,5 mg  Magnesiumstearat

  225,0 mg

Pharmakologische Beobachtungen

Für die Pilotversuche an graviden Meerschweinchen wurden das Prostaglandin Sulproston, das Antigestagen 11ß-[(4-N,N-Dimethylamino)-phenyl]-17ß-hydroxy-17α-(3-hydroxy-prop-1-(Z)-enyl)-4,9(10)-estradien-3-on und als Glukokortikoid Dexamethason als Modellsubstanzen ausgewählt.

Versuchsbeschreibung

Gravide Meerschweinchen mit einem Körpergewicht von ca. 800 g wurden am 42. Tag der Schwangerschaft in den Versuch genommen (der zweite Tag der Vaginalöffnung in der Anpaarungsphase wurde als erster Tag der Gravidität gerechnet). Vor Versuchsbeginn wurde die Gravidität durch Palpation kontrolliert. Die Behandlung mit den Kombinationen erfolgte durch tägliche Injektion am 43. und 44. Tag der Gravidität. Die Testsubstanzen wurden dazu in Benzylbenzoat + Rizinusöl (Mischungsverhältnis bei Sulproston: 1 + 9; Antigestagen 2 + 4,5; Glukokortikoid 2 + 2,4) gelöst und die tägliche Dosis in einem Volumen von 0,4 ml (Sulproston) bzw. 0,5 ml Antigestagen bzw. 0,5 ml Glukokortikoid s.c. injiziert. Das eventuelle Ausstoßen von Früchten wurde unter und nach der Behandlung mehrfach täglich kontrolliert. Am 50. Tag der Gravidität wurden die Tiere getötet. Die Uteri wurden inspiziert und die Foeten befundet.

AG/PG - Kombination

Die Kombination von 10,0 mg /d s.c. 11$\beta$-[(4-N,N-Dimethylamino)-phenyl]-17$\alpha$-hydroxy-17$\beta$-(3-hydroxypropyl)-13$\alpha$-methyl-4,9-gonadien-3-on mit einer marginal wirksamen Sulprostondosis von 0,03 mg/d s.c. führte nach 24 Stunden zur Unterbrechung einer bestehenden Schwangerschaft bei ca. 50% der behandelten Tiere. Mit 10,0 mg 11$\beta$-[(4-N,N-Dimethylamino)-phenyl]-17$\beta$-hydroxy-17$\alpha$- (3-hydroxyprop-1-(Z)-enyl)-4,9(10)-estradien-3-on als Antigestagen beträgt der entsprechende Wert 0%.
Die mit der AG/PG-Kombination bei zweimaliger Injektion erzielten Aborte erfolgten mit einer Latenz von 1 bis 5 Tagen vom Behandlungsbeginn.

AG/PG/GK - Kombination

Die Kombination von 10,0 mg/d s.c. 11$\beta$-[(4-N,N-Dimethylamino)-phenyl]-17$\alpha$-hydroxy-17$\beta$-(3-hydroxypropyl)-13$\alpha$-methyl-4,9-gonadien-3-on, 0,3 mg/ds.c. Dexamethason und 0,03 mg/d s.c. Sulproston führte innerhalb von weniger als 24 Stunden, d. h. bei nur einmaliger Injektion, zu einer Abortrate von 100% (6/6).
Mit 10,0 mg 11$\beta$-[(4-N,N-Dimethylamino)-phenyl]-17$\beta$-hydroxy-17$\alpha$-(3-hydroxyprop-1-(Z)-enyl)-4,9(10)-estradien-3-on als Antigestagen erzielte man eine deutlich höhere Abortrate nach 24 Stunden (60-70% im Vergleich zu 0% bei der entsprechenden AG/PG-Kombination). Außerdem wurde die Latenzzeit deutlich verkürzt (0,5 bis 2,5 Tage).

**Ansprüche**

1. Pharmazeutisches Mittel enthaltend ein Prostaglandin (PG), ein Antigestagen (AG) und ein Glukokortikoid (GK).

2. Pharmazeutisches Mittel gemäß Anspruch 1, dadurch gekennzeichnet, daß Prostaglandin und Antigestagen in einem Gewichtsverhältnis 1 : 10 bis 1 : 10000 stehen.

3. Pharmazeutisches Mittel gemäß Anspruch 1, dadurch gekennzeichnet, daß Prostaglandin und Glukokortikoid in einem Gewichtsverhältnis von 1 : 10 bis 1 : 3000 stehen.

4. Pharmazeutisches Mittel gemäß Anspruch 1, dadurch gekennzeichnet, daß Prostaglandin, Antigestagen und Glukokortikoid in getrennten Dosiseinheiten vorliegen.

5. Pharmazeutisches Mittel gamäß Anspruch 1, dadurch gekennzeichnet, daß Prostaglandin, Antigestagen und Glukokortikoid zusammen in einer Dosiseinheit vorliegen.

6. Pharmazeutisches Mittel gemäß Anspruch 1, dadurch gekennzeichnet, daß eine PG-Dosiseinheit 0,02 - 0,5 mg Sulproston (16-Phenoxy-$\omega$-17,18,19,20-tetranor-PGE$_2$-methylsulfonylamid) oder eine biologisch äquivalente Menge eines anderen Prostaglandins enthält.

7. Pharmazeutisches Mittel gemäß Anspruch 1, dadurch gekennzeichnet, daß eine AG-Dosiseinheit 5 -

200 mg 11$\beta$-[(4--N,N-Dimethylamino)-phenyl]-17$\beta$-hydroxy-17$\alpha$-(3-hydroxy-prop-1(Z)-enyl)-4,9(10)-estradien-3-on oder eine biologisch äquivalente Menge eines anderen Antigestagens enthält.

8. Pharmazeutisches Mittel gemäß Anspruch 1, dadurch gekennzeichnet, daß eine AG-Dosiseinheit 5 - 200 mg 11$\beta$-[(4-N,N-Dimethylamino)-phenyl]-17$\alpha$-hydroxy-17$\beta$-(3-hydroxypropyl)-13$\alpha$-methyl-4,9-gonadien-3-on enthält.

9. Pharmazeutisches Mittel gemäß Anspruch 1, dadurch gekennzeichnet, daß eine GK-Dosiseinheit 0,3 bis 10 mg Dexamethason oder eine biologisch äquivalente Menge eines anderen Glukokortikoids enthält.

10. Verwendung von Prostaglandinen, Antigestagenen und Glukokortikoiden zur Herstellung von Mitteln zur gleichzeitigen getrennten oder zeitlich abgestuften Anwendung zur Geburtseinleitung oder für den Schwangerschaftsabbruch.

11. Verwendung von in einer Dosiseinheit vorliegenden Prostaglandinen, Antigestagenen und Glukokortikoiden zur Herstellung von Mitteln zur Anwendung bei der Geburtseinleitung oder für den Schwangerschaftsabbruch.

12. Verwendung nach Anspruch 10 oder 11, dadurch gekennzeichnet, daß Prostaglandin und Antigestagen in einem Gewichtsverhältnis 1:10 bis 1: 10000 stehen.

13. Verwendung nach Anspruch 10 oder 11, dadurch gekennzeichnet, daß Prostaglandin und Glukokortikoid in einem Gewichtsverhältnis von 1:10 bis 1:3000 stehen.

14. Verwendung nach Anspruch 10 oder 11, dadurch gekennzeichnet, daß man 0,02 - 0,5 mg Sulproston als Prostaglandin oder eine biologisch äquivalente Menge eines anderen Prostaglandins verwendet.

15. Verwendung nach Anspruch 10 oder 11, dadurch gekennzeichnet, daß man 5 - 200 mg 11$\beta$-[(4-N,N-Dimethylamino)-phenyl]-17$\beta$-hydroxy-17$\alpha$-(3-hydroxyprop-1(Z)-enyl-4,9(10)-estradien-3-on als Antigestagen oder eine biologisch äquivalente Menge eines anderen Antigestagens verwendet.

16. Verwendung nach Anspruch 10 oder 11, dadurch gekennzeichnet, daß man 5-200 mg 11$\beta$-[(4-N,N-Dimethylamino)phenyl]-17$\alpha$-hydroxy-17$\beta$-(3-hydroxypropyl)-13$\alpha$-methyl-4,9-gonadien-3-on als Antigestagen verwendet.

17. Verwendung nach Anspruch 10 oder 11, dadurch gekennzeichnet, daß man von von 0,3 bis 10 mg Dexamethason als Glukokortikoid oder einer biologisch äquivalenten Menge eines anderen Glukokortikoids verwendet.

## Claims

1. Pharmaceutical agent containing a prostaglandin (PG), an antigestagen (AG) and a glucocorticoid (GC).

2. Pharmaceutical agent according to claim 1, characterised in that prostaglandin and antigestagen are in a weight ratio of from 1:10 to 1:10000.

3. Pharmaceutical agent according to claim 1, characterised in that prostaglandin and glucocorticoid are in a weight ratio of from 1:10 to 1:3000.

4. Pharmaceutical agent according to claim 1, characterised in that prostaglandin, antigestagen and glucocorticoid are in separate dosage units.

5. Pharmaceutical agent according to claim 1, characterised in that prostaglandin, antigestagen and glucocorticoid are together in one dosage unit.

6. Pharmaceutical agent according to claim 1, characterised in that a PG dosage unit contains from 0.02 to 0.5 mg of sulprostone (16-phenoxy-$\omega$-17,18,19,20-tetranor-PGE$_2$-methylsulfonylamide) or a biologi-

EP 0 179 421 B1

7. Pharmaceutical agent according to claim 1, characterised in that an AG dosage unit contains from 5 to 200 mg of 11β-[(4-N,N-dimethylamino)-phenyl]-17β-hydroxy-17α-(3-hydroxyprop-1(Z)-enyl)-4,9(10)-oestradien-3-one or a biologically equivalent amount of a different antigestagen.

8. Pharmaceutical agent according to claim 1, characterised in that an AG dosage unit contains from 5 to 200 mg of 11β-[(4-N,N-dimethylamino)-phenyl]-17α-hydroxy-17β-(3-hydroxypropyl)-13α-methyl-4,9-gonadien-3-one.

9. Pharmaceutical agent according to claim 1, characterised in that a GC dosage unit contains from 0.3 to 10 mg of dexamethasone or a biologically equivalent amount of a different glucocorticoid.

10. Use of prostaglandins, antigestagens and glucocorticoids for the manufacture of agents for use simultaneously separately or sequentially for inducing labour or for terminating pregnancy.

11. Use of prostaglandins, antigestagens and glucocorticoids in a dosage unit for the manufacture of agents for use in inducing labour or for terminating pregnancy.

12. Use according to claim 10 or claim 11, characterised in that prostaglandin and antigestagen are in a weight ratio of from 1:10 to 1:10000.

13. Use according to claim 10 or claim 11, characterised in that prostaglandin and glucocorticoid are in a weight ratio of from 1:10 to 1:3000.

14. Use according to claim 10 or claim 11, characterised in that there is used as prostaglandin from 0.02 to 0.5 mg of sulprostone or a biologically equivalent amount of a different prostaglandin.

15. Use according to claim 10 or claim 11, characterised in that there are used as antigestagen from 5 to 200 mg of 11β-[(4-N,N-dimethylamino)-phenyl]-17β-hydroxy-17α-(3-hydroxyprop-1(Z)-enyl)-4,9(10)-oestradien-3-one or a biologically equivalent amount of a different antigestagen.

16. Use according to claim 10 or claim 11, characterised in that from 5 to 200 mg of 11β-[(4-N,N-dimethylamino)-phenyl]-17α-hydroxy-17β-(3-hydroxypropyl)-13α-methyl-4,9-gonadien-3-one are used as antigestagen.

17. Use according to claim 10 or claim 11, characterised in that there are used as glucocorticoid from 0.3 to 10 mg of dexamethasone or a biologically equivalent amount of a different glucocorticoid.

**Revendications**

1. Produit pharmaceutique qui contient une prostaglandine (PG), un antiprogestatif (AP) et un glucocorticoïde (GC).

2. Produit pharmaceutique selon la revendication 1 caractérisé en ce que le rapport pondéral de la prostaglandine à l'antiprogestatif est compris entre 1:10 et 1:10 000.

3. Produit pharmaceutique selon la revendication 1 caractérisé en ce que le rapport pondéral de la prostaglandine à l'antiprogestatif est compris entre 1:10 et 1:3000.

4. Produit pharmaceutique selon la revendication 1 caractérisé en ce que la prostaglandine, l'antiprogestatif et le glucocorticoïde sont présentés sous la forme d'unités de prise séparées.

5. Produit pharmaceutique selon la revendication 1 caractérisé en ce que la prostaglandine, l'antiprogestatif et le glucocorticoïde sont réunis dans une même unité de prise.

6. Produit pharmaceutique selon la revendication 1 caractérisé en ce qu'une unité de prise de PG contient

10

de 0,02 à 0,5 mg de sulproston (méthylsulfonylamide de la phénoxy-16-ω-tétranor-17,18,19,20 PGE₂) ou une quantité biologiquement équivalente d'une autre prostaglandine.

7. Produit pharmaceutique selon la revendication 1 caractérisé en ce qu'une unité de prise d'AP contient de 5 à 200 mg de (diméthylamino-4 phényl)-11β hydroxy-17β [hydroxy-3 propène-1(Z) yl]-17α estradiène-4,9(10) one-3 ou une quantité biologiquement équivalente d'un autre antiprogestatif.

8. Produit pharmaceutique selon la revendication 1 caractérisé en ce qu'une unité de prise d'AP contient de 5 à 200 mg de (diméthylamino-4 phényl)-11β hydroxy-17α (hydroxy-3 propyl)-17β méthyl-13α gonadiène-4,9 one-3.

9. Produit pharmaceutique selon la revendication 1 caractérisé en ce qu'une unité de prise de GC contient de 0,3 à 10 mg de dexaméthazone ou une quantité biologiquement équivalente d'un autre glucocorticoïde.

10. Application de prostaglandines, d'antiprogestatifs et de glucocorticoïdes pour la fabrication de produits destinés à être appliqués en même temps, séparément ou graduellement dans le temps, pour le déclenchement de l'accouchement ou pour l'interruption de la grossesse.

11. Application de prostaglandines, d'antiprogestatifs et de glucocorticoïdes se trouvant en une unité de prise pour la fabrication de produits destinés à être appliqués pour le déclenchement de l'accouchement ou pour l'interruption de la grossesse.

12. Application selon l'une des revendications 10 et 11, caractérisée en ce que le rapport pondéral de la prostaglandine à l'antiprogestatif est compris entre 1:10 et 1:10 000.

13. Application selon l'une des revendications 10 et 11, caractérisée en ce que le rapport pondéral de la prostaglandine au glucocorticoïde est compris entre 1:10 et 1:3000.

14. Application selon l'une des revendications 10 et 11, caractérisée en ce qu'on utilise de 0,02 à 0,5 mg de sulproston comme prostaglandine ou une quantité biologiquement équivalente d'une autre prostaglandine.

15. Application selon l'une des revendications 10 et 11, caractérisée en ce qu'on utilise de 5 à 200 mg de (diméthylamino-4 phényl)-11β hydroxy-17β [hydroxy-3 propène-1(Z) yl]-17α estradiène-4,9(10) one-3 comme antiprogestatif ou une quantité biologiquement équivalente d'un autre anti-progestatif.

16. Application selon l'une des revendications 10 et 11, caractérisée en ce qu'on utilise de 5 à 200 mg de-(diméthylamino-4 phényl)-11β hydroxy-17α (hydroxy-3 propyl)-17β méthyl-13α gonadiène-4,9 one-3 comme antiprogestatif.

17. Application selon l'une des revendications 10 et 11, caractérisée en ce qu'on utilise de 0,3 à 10 mg de dexaméthazone comme glucocorticoïde ou une quantité biologiquement équivalente d'un autre gluco-corticoïde.